# EUROPEAN PATENT APPLICATION

(11) **EP 4 809 990 A1**
(43) Date of publication of application: **23.09.2026**
(21) Application number: 26166506.1
(22) Date of filing: 20.03.2026
(51) Int. Cl.: A61F 9/06, H05K 5/06, H01H 9/04

(54) **WELDING HELMET AND AUTO-DARKENING FILTER THEREFOR**

(30) Priority: 20.03.2025 CN 202510336460
(71) Applicant: TECMEN ELECTRONICS CO., LTD, Nanjing 211500 (CN)
(72) Inventor: WEI, Kailin, Jiangsu, 211500 (CN)
(74) Representative: Lippert Stachow Patentanwälte Rechtsanwälte

(57) **Abstract**

The present disclosure relates to an auto-darkening filter for a welding helmet, comprising an outer shell, a control circuit (20), an auto-darkening panel and an adjustment assembly; the outer shell (10) including a first shell body (11) and a second shell body (12) provided oppositely to each other, wherein when the auto-darkening filter is installed on the welding helmet, the first shell body faces towards a user's face, the first shell body has an annular projection (111) protruding from its outer surface, the annular projection is used to block liquid entering the outer shell, both of the control circuit and the auto-darkening panel are disposed in an interior of the outer shell, the auto-darkening filter is electrically connected to the control circuit; the adjustment knob (31) is used for the user to the adjust various operating parameters of the auto-darkening filter; sweat when generating from the user will flow on the outer surface of the first shell body and cannot enter the outer shell via the annular projection to avoid corrosion and damage of electronic components inside the auto-darkening filter due to liquid ingress. The present disclosure also relates to a welding helmet comprising a helmet body and the auto-darkening filter.

## Description

### Field

The present disclosure generally relates to the technical field of a welding protection apparatus, and more particularly to a welding helmet and an auto-darkening filter therefor.

### Background

An auto-darkening welding helmet is a welding protection apparatus to provide welding workers a clear field of vision before he or she makes a welding operation, facilitating precise positioning of welding points and avoiding unexpected blind welding and bare welding. When a welding arc occurs, an internal photoelectric sensing circuit of the auto-darkening welding helmet can detect arc light such that a control circuit of a liquid crystal panel can be triggered such that liquid crystal changes its light transmittance according to a preset light transmittance and thus the arc light is allowed to pass through the liquid crystal panel at an intensity weak enough for human eyes to tolerate. In the meanwhile, any harmful rays such as infrared or ultraviolet rays can be filtered out.

An auto-darkening filter is a core element of the auto-darkening welding helmet, which can be switchable very quickly from a clear transparent state to a darkness state in which state human eyes are prevented from being harmed by sudden strong light. Usually, the auto-darkening filter has functional knobs for parameter adjustment such as a knob for shading adjustment, a knob for sensitivity adjustment, a knob for delay adjustment or the like.

During a welding process, a large amount of heat can be generated and therefore welding works are exposed on a high-temperature environment. To maintain a normal body temperature, human body dissipates heat through sweating. Therefore, the welding workers tend to sweat a lot during the welding process. Besides, the welding operation usually requires that the welding workers to maintain high concentration and a specific working posture for a long time period, leading to a heavier physical load on the welding workers and making them more prone to sweating. Due to the reasons mentioned above, the welding works' sweat is prone to entering an interior of the auto-darkening filter, causing circuit corrosion therein and affecting the service life and performance of the auto-darkening filter. Therefore, there is an urgent need to improve and optimize the existing auto-darkening filter.

### Summary

In order to solve the technical issues already mentioned, the present disclosure proposes an auto-darkening filter for a welding helmet, comprising: an outer shell including a first shell body and a second shell body provided oppositely to each other, wherein when the auto-darkening filter is installed on the welding helmet, the first shell body faces towards a user's face, the first shell body has at least an annular projection protruding from its outer surface; a control circuit disposed in an interior of the outer shell; an auto-darkening panel disposed in the interior of the outer shell and electrically connected to the control circuit; at least one adjustment assembly including an adjustment knob and a potentiometer, the adjustment knob is at least partially exposed on the outer surface of the first shell body and is covered about an outer periphery of the annular projection, the potentiometer is disposed in the interior of the outer shell and is electrically connected to the control circuit, the potentiometer is also coupled to the adjustment knob such that when the adjustment knob is rotated, it can act on the potentiometer.

In some embodiments of the present disclosure, the adjustment knob comprises:
an outer knob in the form of a cap and at least partially exposed on the outer surface of the first shell body and be covered about the outer periphery of the annular projection;
an inner knob disposed in the interior of the outer shell, one side of the inner knob adjacent to the outer knob being connected to the outer knob such that coaxial rotation of the inner knob is driven by the outer knob; the other side of the inner knob away from the outer knob having a rotational shaft coupled to the potentiometer.

In some embodiments of the present disclosure, one of the outer and inner knobs has at least a positioning projection, the other of the outer and inner knobs has at least a positioning groove mating with the positioning projection, such that the positioning projection is insertable and securable in the positioning groove.

In some embodiments of the present disclosure, the positioning groove is disposed in the inner knob and the positioning groove is embodied as multiple positioning grooves. That is, there are multiple positioning grooves disposed in the inner knob.

In some embodiments of the present disclosure, the inner knob at its outer contour is provided with a first limit part protruding therefrom, the first shell body at its inner surface is provided with a second limit part corresponding to the first limit part, when the first limit part is in contact with the second limit part, further rotation of the inner knob is impeded by the second limit part.

In some embodiments of the present disclosure, a side of the adjustment knob adjacent to the annular projection is provided with an annular groove for mating with the annular projection, such that the annular projection can be received in the annular groove.

In some embodiments of the present disclosure, the first shell body also comprises a first engagement part disposed on a circumferential inner wall of the annular projection and extending along a radial direction of the annular projection towards the center of the annular projection;
wherein the adjustment knob also comprises a second engagement part disposed adjacent to the annular groove, when the adjustment knob is installed, the second engagement part is snapped with the first engagement part.

In some embodiments of the present disclosure, a side of the adjustment knob adjacent to the potentiometer is provided with a rotational shaft coupled to the potentiometer, an isolation part is formed in a portion between the rotational shaft and the annular groove, and the second engagement part is disposed at a side of the isolation part adjacent to the annular groove.

In some embodiments of the present disclosure, the adjustment knob also comprises a first limit part disposed in the annular groove, the first shell body also comprises a second limit part disposed on a top of the annular projection and for mating with the first limit part, when the first limit part is in contact with the second limit part, further rotation of the adjustment knob is impeded by the second limit part.

The present disclosure also proposes a welding helmet, comprising a helmet body and an auto-darkening filter as mentioned above, wherein the helmet body can be worn by a head of a user, and the auto-darkening filter is releasably connected to the helmet body.

Benefits of the present disclosure: The present disclosure relates to an auto-darkening filter for a welding helmet, comprising an outer shell, a control circuit, an auto-darkening panel and an adjustment assembly; the outer shell including a first shell body and a second shell body provided oppositely to each other, wherein when the auto-darkening filter is installed on the welding helmet, the first shell body faces towards a user's face, the first shell body has an annular projection protruding from its outer surface, the annular projection is used to block liquid entering the outer shell, both of the control circuit and the auto-darkening panel are disposed in an interior of the outer shell, the auto-darkening filter is electrically connected to the control circuit; the adjustment knob is used for the user to the adjust various operating parameters of the auto-darkening filter; sweat when generating from the user will flow on the outer surface of the first shell body and cannot enter the outer shell via the annular projection to avoid corrosion and damage of electronic components inside the auto-darkening filter due to liquid ingress. The present disclosure also relates to a welding helmet comprising a helmet body and the auto-darkening filter.

### Brief description to the drawings

The foregoing and additional aspects, features, and advantages of the present disclosure will become more readily apparent from the following detailed description of embodiments, depicted in the attached drawings, in which:
Fig. 1A is a front view schematically illustrating an auto-darkening filter of a welding helmet according to an embodiment of the present disclosure;
Fig. 1B is a rear view schematically illustrating the auto-darkening filter of the welding helmet according to the embodiment of the present disclosure;
Fig. 1C is a side view schematically illustrating the auto-darkening filter of the welding helmet according to the embodiment of the present disclosure;
Fig. 1D is a diagram schematically illustrating, from one perspective, the auto-darkening filter of the welding helmet according to the embodiment of the present disclosure;
Fig. 1E is a diagram schematically illustrating, from another perspective, the auto-darkening filter of the welding helmet according to the embodiment of the present disclosure;
Fig. 1F is a diagram schematically illustrating, from two perspectives, an outer knob in the auto-darkening filter of the welding helmet according to the embodiment of the present disclosure;
Fig. 1G is a diagram schematically illustrating, from two perspectives, an inner knob in the auto-darkening filter of the welding helmet according to the embodiment of the present disclosure;
Fig. 1H is a rear view schematically illustrating the auto-darkening filter of the welding helmet according to the embodiment of the present disclosure;
Fig. 1I is a cross-sectional view obtained along A-A of Fig. 1H;
Fig. 2 is a partial and cross-sectional view schematically illustrating an auto-darkening filter of a welding helmet according to another embodiment of the present disclosure;
Fig. 3A is a rear view schematically illustrating the auto-darkening filter of the welding helmet according to another embodiment of the present disclosure;
Fig. 3B is a cross-sectional view obtained along E-E of Fig. 3A;
Fig. 3C is a diagram schematically illustrating, from two perspectives, an adjustment knob adopted in another embodiment of the auto-darkening filter of the welding helmet;
Fig. 3D is a diagram schematically illustrating, from one perspective, a first shell body adopted in another embodiment of the auto-darkening filter of the welding helmet;
Fig. 3E is a diagram schematically illustrating, from another perspective, the first shell body adopted in another embodiment of the auto-darkening filter of the welding helmet;
Fig. 4 is a perspective view schematically illustrating a welding helmet according to an embodiment of the present disclosure.

### List of reference numerals:

- 100: auto-darkening filter
- 10: outer shell
- 11: first shell body
- 111: annular projection
- 112: second limit part
- 113: first engagement part
- 114: range mark
- 115: installation hole
- 12: second shell body
- 20: control circuit
- 30: adjustment assembly
- 31: adjustment knob
- 311: outer knob
- 3111: friction increasing feature
- 3112: indicator mark
- 312: inner knob
- 312a: first knob part
- 312b: second knob part
- 3121: rotational shaft
- 313: positioning protrusion
- 314: positioning groove
- 315: first limit part
- 316: annular groove
- 317: second engagement part
- 319: isolation part
- 32: potentiometer
- 40: solar panel
- 50: sealing element
- 200: welding helmet

### Embodiments

Example embodiments are described below with reference to the accompanying drawings. Unless otherwise expressly stated, the sizes, positions, etc., of components, features, elements, etc., as well as any distances therebetween, are not necessarily to scale, and may be disproportionate and/or exaggerated for clarity.

The embodiments described herein are merely examples, set forth by way of illustration only and not limitation. Those skilled in the art will recognize in light of the teachings herein that there are alternatives, variations and equivalents to the example embodiments described herein and their component parts. For example, other embodiments are readily possible, variations can be made to the embodiments described herein, and there may be equivalents to the components, parts, or steps that make up the described embodiments.

For the sake of clarity and conciseness, certain aspects of components or steps of certain embodiments are presented without undue detail where such detail would be apparent to those skilled in the art in light of the teachings herein and/or where such detail would obfuscate an understanding of more pertinent aspects of the embodiments.

The terminology used herein is for the purpose of describing particular example embodiments only and is not intended to be limiting. As used herein, the singular forms "a", "an" and "the" are intended to include the plural forms as well, unless the context clearly indicates otherwise. It should be recognized that the terms "comprise", "comprises", "comprising", "include", "includes", "including", "has", "have", and "having", when used in this document, are open-ended and specify the presence of stated features, integers, steps, operations, elements, and/or components, but do not preclude the presence or addition of one or more other features, integers, steps, operations, elements, components, and/or groups thereof. Unless otherwise specified, a range of values, when recited, includes both the upper and lower limits of the range, as well as any sub-ranges therebetween.

Unless indicated otherwise, the terms "about", "thereabout", "substantially", etc. mean that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art.

Unless clearly indicated otherwise, all functional or operative connections may be direct or indirect. Similarly, unless clearly indicated otherwise, all physical connections may be rigid or non-rigid, permanent or temporary, direct or indirect (e.g., via intermediary components).

Like numbers refer to like elements throughout. Thus, the same or similar numbers may be described with reference to other drawings even if they are neither mentioned nor described in the corresponding drawing. Also, even elements that are not denoted by reference numbers may be described with reference to other drawings. Additionally, the drawings may include non-essential elements that are included only for the sake of thoroughness. These non-essential elements may be removed entirely or left only in outline form if drawing changes are desired to create greater clarity.

Not every feature shown in every drawing is labeled with a reference number, even though the same feature may be labeled with a reference number on other drawings. Reference numbers have been omitted where it is believed they would unnecessarily clutter a drawing. However, all rights are reserved to add reference numbers to the drawings to clarify aspects of the embodiments. Moreover, some views omit some features shown in other views. Finally, the drawings sometimes illustrate variations from one drawing to another, even where those drawings are intended to depict the same embodiment.

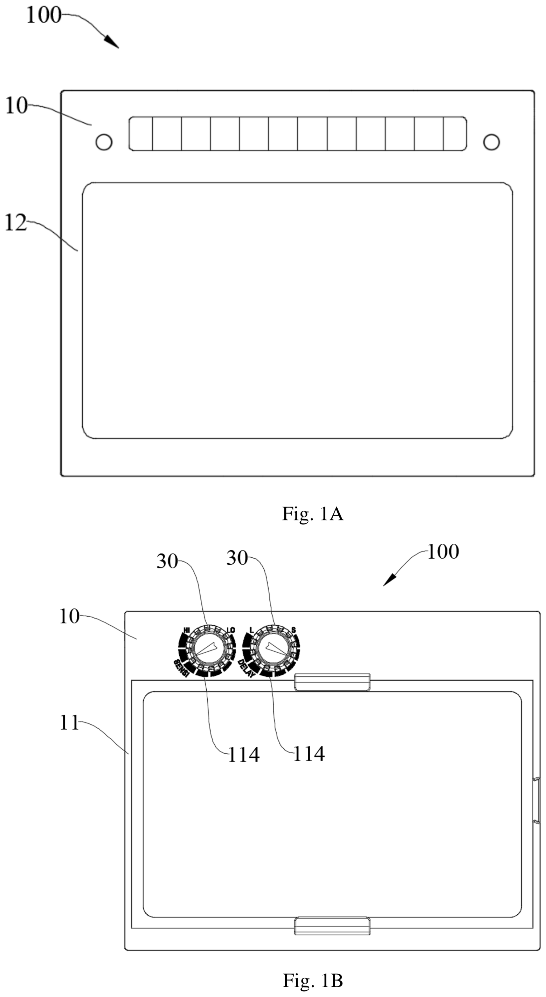

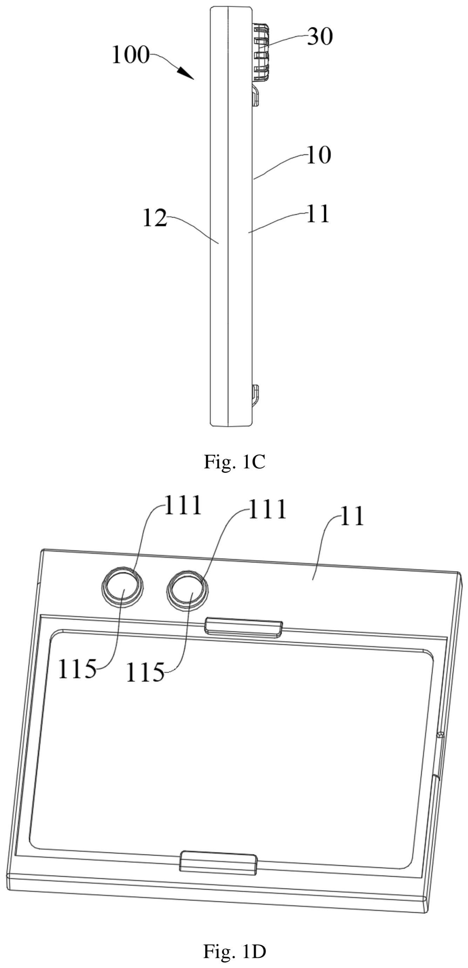

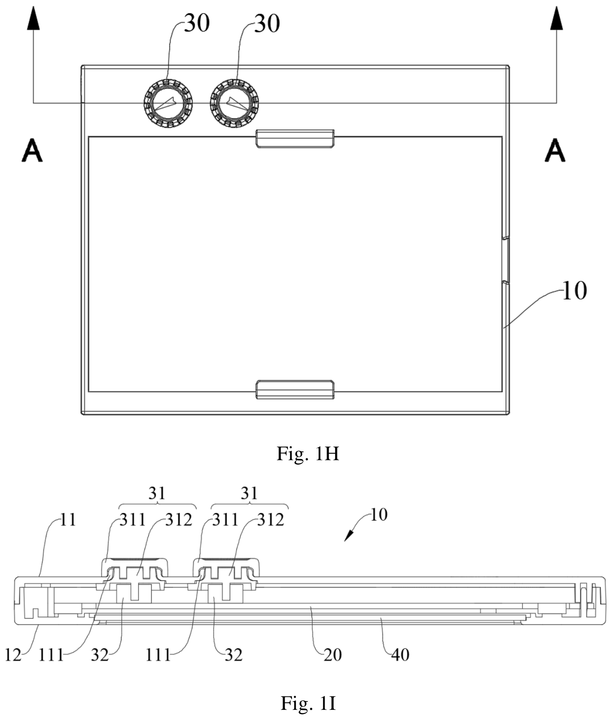

In an embodiment as shown by Figs. 1A to 1I, an auto-darkening filter 100 is used for a welding helmet 200, and the auto-darkening filter 100 comprises an outer shell 100, a control circuit 20, an auto-darkening panel and at least one adjustment knob 30.

The outer shell 10 comprises a first shell body 11 and a second shell body 12 provided oppositely to each other or relative to each other. A relatively closed space is enclosed by the first shell body 11 and the second shell body 12. When the auto-darkening filter 100 is fitted onto the welding helmet 200 in place, the first shell body 11 faces towards a user's face. That is to say, the first shell body 11 is proximal to the user's face. Especially, when the welding helmet is worn on the user's head, the first shell body 11 will face towards the user's face. As shown by Fig. 1D, the first shell body 11 has at least an installation hole 115 through which at least an adjustment knob 30 can be fitted onto the outer shell 10. The first shell body 11 has at least an annular projection 111 protruding from its outer surface. The projection 111 is provided about the respective installation hole 115. The annular projection 111 is used to prevent liquids (such as the user's sweat) from entering the outer shell 10, thereby avoiding corrosion and damage of electronic components inside the auto-darkening filter 100 due to liquid ingress.

Furthermore, the control circuit 20 is used to control the entire auto-darkening filter 100, and is disposed in an interior of the outer shell 10. The auto-darkening panel (not shown here) is switchable between a clear transparent state and a darkness state, is disposed in the interior of the outer shell 10 and is electrically connected to the control circuit 20. The adjustment assembly 30 can be used by a user to adjust various parameters of the auto-darkening filter 100, such as SENSITIVITY (a response rate at which the auto-darkening panel is switched from the clear transparent state to the darkness state), DELAY RATE/DELAY (a response rate at which the auto-darkening panel is switched from the darkness state to the clear transparent state) or the like. Each adjustment assembly 30 can be configured to adjust one parameter of the auto-darkening filter 100 correspondingly.

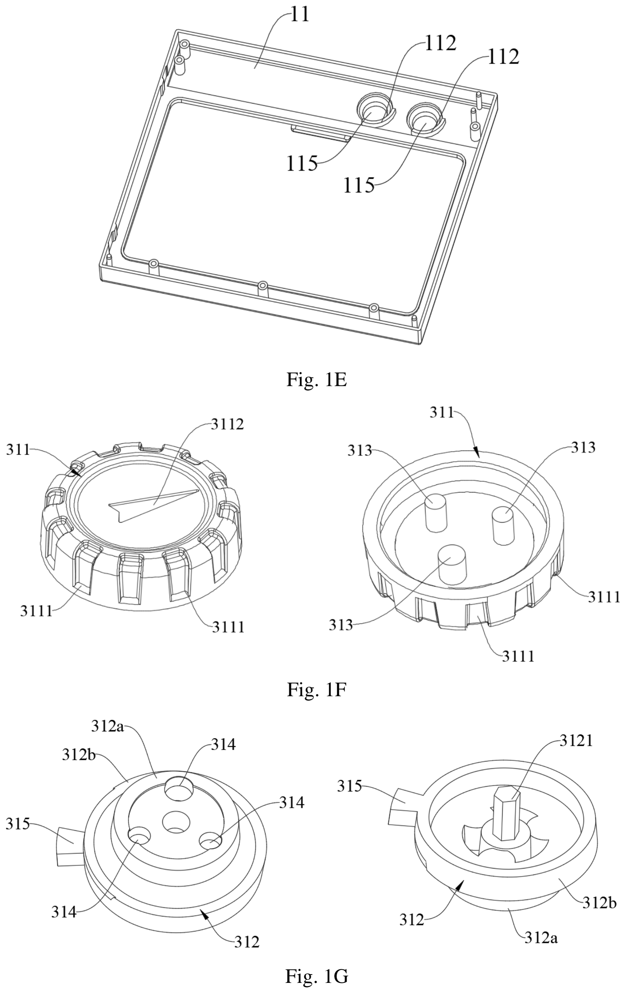

As shown inf Figs. 1F to 1I, the adjustment assembly 30 comprises an adjustment knob 31 and a potentiometer 32. The adjustment knob 31 is at least exposed on the outer surface of the first shell body 11 and is covered about an outer periphery of the annular projection 111. The potentiometer 32 is disposed in the interior of the outer shell 10 and is electrically connected to the control circuit 20. The potentiometer 32 is also coupled to the adjustment knob 31 such that when the adjustment knob 31 is rotated, it (or the rotation) can act on the potentiometer 32. The potentiometer 32 is configured to transmit signals to the control circuit 20 and just the parameters of the auto-darkening filter 100 via the control circuit 20.

As shown by Fig. 1D, the annular projection 111 is configured to protrude from the outer surface of the first shell body 11. After sweat is generated by a user, the sweat will flow on the outer surface of the first shell body 11, and cannot enter the outer shell 10 through the annular projection 111, to avoiding corrosion and damage of electronic components inside the auto-darkening filter 100 due to liquid ingress. Besides, the annular projection 111 is provided such that it is not in contact with an inner wall of the adjustment knob 31 or if there is frictional resistance between the annular projection 111 and the adjustment knob 31 due to contact therebetween, the frictional resistance is such small that rotation of the adjustment knob 31 cannot be affected by the frictional resistance. Moreover, since the adjustment knob 31 is covered about the outer periphery of the annular projection 111, the annular projection 111 can be protected correspondingly. The adjustment knob 31 has a side adjacent to the outer surface of the first shell body 11. There is a small gap between the side of the adjustment knob 31 and the outer surface of the first shell body 11, further preventing liquids from entering the interior of the outer shell 10 and resulting in enhanced waterproof performance of the auto-darkening filter 100. When a user rotates the adjustment knob 31 with a wet hand, liquids will not enter the interior of the outer shell 10.

Furthermore, in the embodiment as shown by Figs. 1F to 1I, the adjustment knob 31 comprises an outer knob 311 and an inner knob 312. The outer knob 311 is generally in the form of a cap, and is configured to be at least partially exposed on the outer surface of the first shell body 11 and be covered about the outer periphery of the annular projection 111.

The inner knob 312 is provided in the outer shell 10. The inner knob 312 is coupled to the outer knob 311 at one side adjacent to the outer knob 311 such that the inner knob 312 can be driven by the outer knob 311 to rotate coaxially. The inner knob 312 is provided with a rotational shaft 3121 on the other side away from the outer knob 311. The rotational shaft 3121 is coupled to the potentiometer 32. When the outer knob 311 is rotated by a user, the inner knob 312 will rotate coaxially together with the outer knob 311 and will act on the potentiometer 32 via the rotational shaft 3121.

In some embodiments, the outer knob 311 and the inner knob 312 can be permanently fixed and connected to each other, such as by welding, gluing, etc. In alternative embodiments, the outer knob 311 and the inner knob 312 can be non-permanently fixed and connected to each other, such as by plugging-in, snapping-in, screw connection, etc., to conveniently assemble or disassemble them.

Furthermore, in the illustrated embodiment, in order to facilitate accurate positioning of the outer knob 311 relative to the inner knob 312 or the inner knob 321 relative to the outer knob 311 during a process of assembling them, one of the outer knob 311 and the inner knob 312 is provided with at least a positioning projection 313, and the other of the outer knob 311 and the inner knob 312 is provided with at least a positioning groove 314 for mating with the positioning projection 313. The positioning projection 313 is insertable and securable in the positioning groove 314 to achieve plugging-in positioning between the outer knob 311 and the inner knob 312. Preferably, the positioning projection 313 is secured in the positioning groove 314 by interference fit. Additionally, the positioning protrusion 313 can also be strengthened and secured in the positioning groove 314 by adhesive bonding.

Furthermore, the positioning projection 313 and the positioning groove 314 can be interchanged at their positions. That is, in some embodiments, the positioning projection 313 can be provided in the outer knob 311, and the positioning groove 314 can be provided in the inner knob 312; in some embodiments, the positioning projection 313 is provided in the inner knob 312 and the positioning groove 314 is provided in the outer knob 313; in some embodiments, both of the outer knob 311 and the inner knob 312 are provided with the positioning projection 313 and the positioning groove 314.

Preferably, in the embodiment as shown by Figs. 1F and 1G, the positioning projection 313 is provided in the outer knob 311 and the positioning groove 314 is provided in the inner knob 312. Both of the outer knob 311 and the inner knob 312 have a circular cross-sectional shape, and thus the positioning projection 313 and the positioning groove 314 are eccentrically provided.

Furthermore, to ensure better stability of the outer knob 311 and the inner knob 312 after they are assembled together, multiple positioning protrusions 313 and multiple positioning grooves 314 are provided and they are regularly distributed. For example, two, three, four or more positioning protrusions 313 and positioning grooves 314 can be provided respectively. Preferably, three positioning protrusions 313 and three positioning grooves 314 are provided respectively. They are regularly distributed in a triangular pattern.

As shown by Fig. 1G, preferably, three positioning grooves 314 are provided such that they are distributed around the center of the inner knob 312.

As shown by Fig. 1B, two adjustment assemblies 30 are schematically provided in the auto-darkening filter 100; but more adjustment assemblies 30 can be alternatively provided in the auto-darkening filter 100. The two adjustment assemblies 30 are configured as a SENSITIVEITY adjusting switch and a DELAY adjusting switch respectively. Two range marks 114 are disposed at locations of the outer surface of the first shell body 11 corresponding to the two adjustment assemblies 30, and are used to indicate various settings of SENSITIVEITY and various setting of DELAY respectively. Correspondingly, as shown by Fig. 1F, there is an indicator mark 3112 provided on a top of the outer knob 311 which is used to align the range mark 114. For example, the indicator mark 3112 can be enabled to align different zones of the range mark 114 to select different settings respectively.

In Fig. 1B, the range mark 114 is generally fan-shaped, and does not occupy or surround the entire circumference of the adjustment knob 31 in its rotational direction. Therefore, in an embodiment of the present disclosure, the adjustment knob 31 is limited to be rotatable in an angular arrange. Specifically, as shown by Fig. 1G, the inner knob 312 at its outer contour is provided with a first limit part 315 protruding therefrom; as shown by Fig. 1E, the first shell body 11 is provided with a second limit part 112 at its inner surface, which second limit part 112 is used to mate with the first limit part 315. When the first limit part 315 is in contact with the second limit part 112, the second limit part 112 will prevent the inner knob 312 from rotating further.

Preferably, the first limit part 315 is in the form of a lug, and the second limit part 112 is in the form of a crescent arc. During rotation of the first limit part 315 together with the inner knob 312, both ends of the second limit part 112 can block the first limit part 315 respectively, thereby constraining the rotation of the inner knob 312 within a limited angular range (for example as defined by the both ends) and restricting any further unexpected rotation of the inner knob 312.

In the embodiment as shown by Fig. 1G, the inner knob 312 is substantially configured as a convex member, and comprises a first knob part 312a and a second knob part 312b along its axial direction. The first knob par 312a is adjacent to the outer knob 311, and the first knob part 312a has a diameter less than that of the first knob part 312b. The positioning groove 314 is provided in the first knob part 312a, and the first limit part 315 is provided in the second knob part 312b.

As shown by Fig. 1F, to enable a user to rotate the outer knob 311 easily, a friction increasing feature 3111 is provided in a surface of the outer knob 311. The friction increasing feature 3111 can be embodied as protrusions, grooves or the like to increase the surface friction of the friction increasing feature 3111.

As shown by Fig. 1I, a solar panel 40 is provided in the outer shell 10. The solar panel 40 can be configure to convert strong light into electrical energy and store it for operating the auto-darkening filter 100 later. Besides, the auto-darkening filter 100 also has a photosensitive element, a power supply or other suitable elements whose explanations can refer to an existing auto-darkening filter and will not be given in the present disclosure.

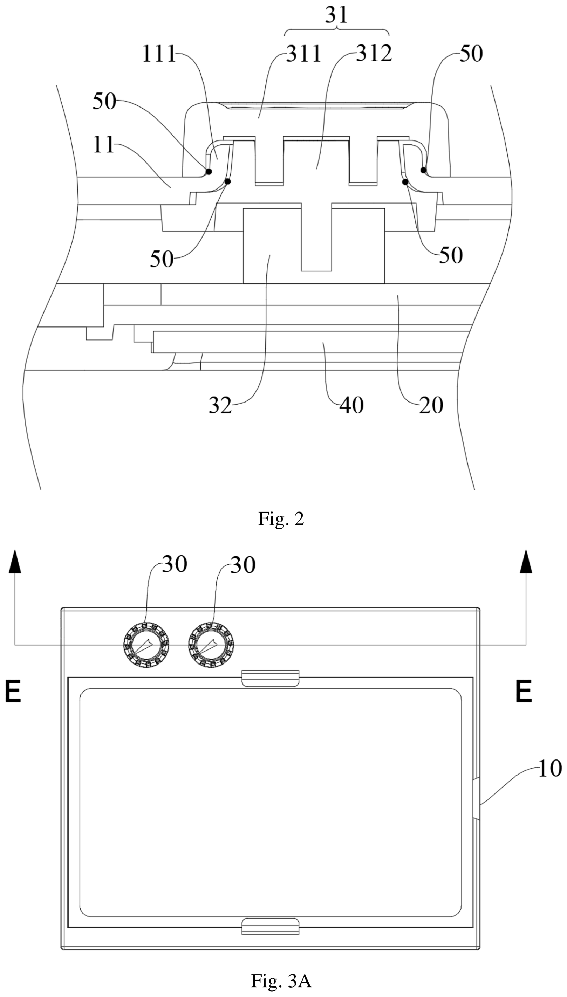

Furthermore, as shown by Fig. 2, in some embodiments of the present disclosure, a sealing element 50 is provided at the junction of the first knob part 312a and the second knob part 312b. For example, the sealing element 50 can be in the form of a sealing ring or the like. The sealing element 50 can be used to further enhance the waterproof performance of the adjustment knob 31, avoid liquid corrosion of electronic components inside the auto-darkening filter 100 without blocking the rotation of the inner knob 312 in any way. In some embodiments of the present disclosure, an additional sealing element 50 can be provided at the junction of the annular projection 111 and the first shell body 11. This sealing element 50 can be used further to prevent sweat from entering the interior of the outer shell 10, without blocking rotation of the outer knob 311 in any way.

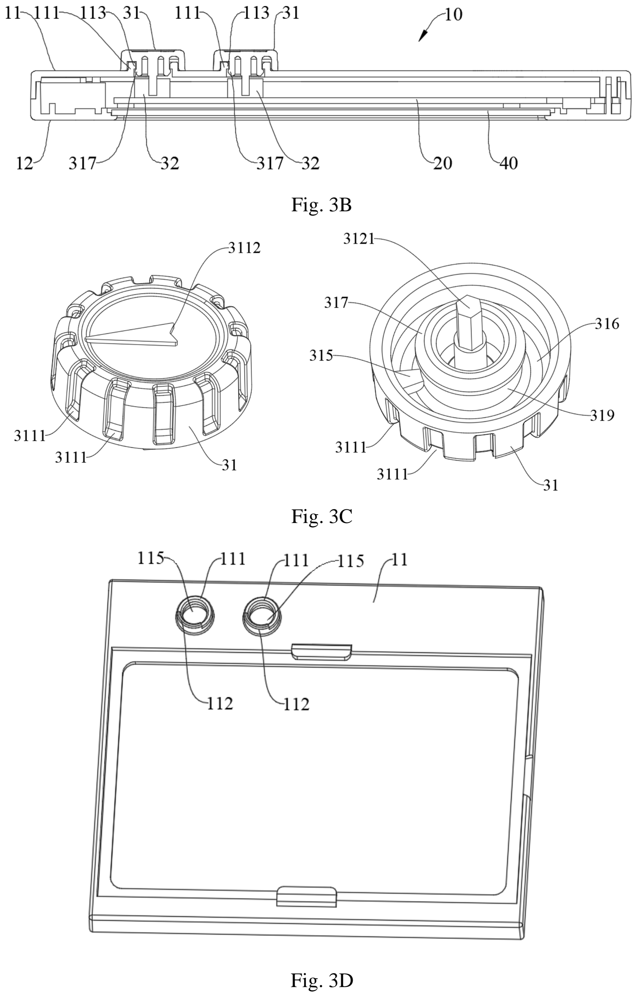

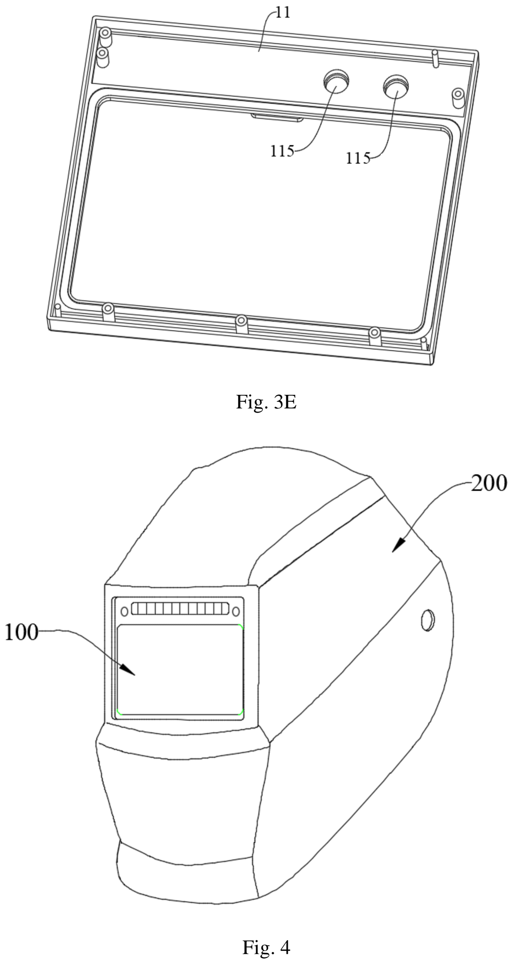

Figs. 3A to 3E shows an alternative embodiment of the auto-darkening filter 100 which is distinguished from the embodiment as shown by Figs. 1A to 1I in that the adjustment knob 30 is differently configured and the position at which the second limit part 112 is provided is also changed.

In the embodiment as shown by Fig. 3C, a side of the adjustment knob 31 adjacent to the annular projection 111 is provided with an annular groove 316 for mating with the annular projection 111. The annular groove 316 has a circular-ringed cross-section such that the annular projection 111 can be received in the annular groove 316.

Furthermore, a side of the adjustment knob 31 adjacent to the potentiometer 32 is provided with a rotational shaft 3121. Preferably, the rotational shaft 3121 is provided at the center of the adjustment knob 31 and is connected to the potentiometer 32.

As shown by Fig. 3B, to avoid detachment of the adjustment knob 31 from the first shell body 11, the first shell body 11 also comprises a first engagement part 113 provided on a circumferential inner wall of the annular projection 111. The first engagement part 113 is configured to extend along a radial direction of the annular projection 111 towards a center of the annular projection 111. Therefore, the shape of the first engagement part 113 is a protruding annular structure. As shown by Fig. 3C, the adjustment knob 31 also comprises a second engagement part 317 provided adjacent to the annular groove 316. The second engagement part 317 is also shaped as a protruding annular structure. When the adjustment knob 31 is installed, the second engagement part 317 is snapped with the first engagement part 113, making it difficult for the second engagement part 317 to go over the first engagement part 113 to avoid detachment of the adjustment knob 31 from the first shell body 11. Moreover, after the adjustment knob 31 is installed in the first shell body 11, there is no contact between the first engagement part 113 and the second engagement part 317 or a very small friction exits therebetween even if they are in contact with each other, such that the rotation of the adjustment knob 31 will not be impeded by the first engagement part 113.

As shown by Figs. 3B and 3C, an isolation part 319 is formed in a portion between the rotational shaft 3121 and the annular groove 316. The second engagement 317 is provided on a side of the isolation part 319 adjacent to the annular groove 316.

Furthermore, as shown by Fig. 3A, the adjustment assembly 30 is schematically designed as two adjustment assemblies 30 in the auto-darkening filter 100, but designing more adjustment assemblies 30 is also feasible. The two adjustment assemblies 30 are used as a SENSITIVITY adjusting switch and a DELAY adjusting switch respectively. Two range marks (not shown in Fig. 3A, but one can refer to Fig. 1B) can be provided at corresponding locations of the first shell body 11, to indicate various settings of SENSITIVEITY and various setting of DELAY respectively. Correspondingly, there is an indicator mark 3112 provided on a top of a respective adjustment knob 31 which is used to align the range mark. The indicator mark 3112 can be enabled to align different zones of the range mark to select different settings respectively.

Furthermore, the range mark is generally fan-shaped, and does not occupy or surround the entire circumference of the adjustment knob 31 in its rotational direction. Therefore, in an embodiment of the present disclosure, the adjustment knob 31 is limited to be rotatable in an angular arrange. Specifically, the adjustment knob 311 also comprises a first limit part 315 provided in the annular groove 316; the first shell body 11 also comprises a second limit part 112 provided on a top of the annular projection 111 and used for mating with the first limit part 315. After the first limit part 315 is in contact with the second limit part 112, further rotation of the adjustment knob 31 is impeded by the second limit part 112.

Preferably, the first limit part 315 is in the form of a lug, and the second limit part 112 is in the form of a crescent arc. During rotation of the first limit part 315 together with the adjustment knob 31, both ends of the second limit part 112 can block the first limit part 315 respectively, thereby constraining the rotation of the adjustment knob 31 within a limited angular range and restricting any further unexpected rotation of the adjustment knob 31.

As shown by Fig. 3C, in order to enable a user to rotate the adjustment knob 31 easily, a friction increasing feature 3111 is provided in a surface of the adjustment knob 31. The friction increasing feature 3111 can be embodied as protrusions, grooves or the like to increase the surface friction of the friction increasing feature 3111.

Furthermore, on the basis of Figs. 3A to 3E, in some embodiments of the present disclosure, an additional sealing element is also provided at the junction of the annular projection 111 and the first shell body 11. For example, the additional sealing element can be in the form of a sealing ring or the like. The sealing element can be used to further prevent sweat from entering the interior of the outer shell 10 without blocking the rotation of the adjustment knob 31 in any way.

Furthermore, the present disclosure also relates to a welding helmet 200 which comprises a helmet body and the auto-darkening filter 100 explained in the embodiments. The helmet body can be worn on a head of a user. The auto-darkening filter 100 is releasably connectable to the helmet body.

Finally, it is hereby clarified that where directional indications (such as up, down, left, right, front, rear, etc.) are referred to in the embodiments of the present application, such directional indications shall only be used to explain the relative positional relationships, movements, etc., among the components under a specific posture (as shown in the attached figures). If such specific posture is changed, such directional indications shall be changed accordingly.

In addition, where descriptions such as "first", "second", etc., are referred to in the embodiments of the present application, such descriptions as "first", "second", etc., shall be used for descriptive purposes only and shall not be construed as indicating or implying their relative importance or implicitly indicating the number of the indicated technical features. Accordingly, a feature defined as "first" or "second" may expressly or implicitly include at least one such feature. Furthermore, the technical solutions among the various embodiments may be combined with each other, provided that such combination is capable of being implemented by a person having ordinary skill in the art. If a combination of technical solutions is mutually contradictory or incapable of being implemented, such combination of technical solutions shall be deemed non-existent and shall not fall within the scope of protection claimed by the present application.

The foregoing are merely the embodiments of the present application and shall not limit the patent scope of the present application. Any equivalent structural or equivalent process transformation made by using the contents of the description and the attached drawings of the present application, or any direct or indirect application thereof in other relevant technical fields, shall be similarly included within the patent protection scope of the present application.

## Claims

1. An auto-darkening filter for a welding helmet, comprising:
an outer shell including a first shell body and a second shell body provided oppositely to each other, wherein when the auto-darkening filter is installed in the welding helmet, the first shell body faces towards a user's face, the first shell body has at least an annular projection protruding from its outer surface;
a control circuit disposed in an interior of the outer shell;
an auto-darkening panel disposed in the interior of the outer shell and electrically connected to the control circuit;
at least one adjustment assembly including an adjustment knob and a potentiometer, the adjustment knob is at least partially exposed on the outer surface of the first shell body and is covered about an outer periphery of the annular projection, the potentiometer is disposed in the interior of the outer shell and is electrically connected to the control circuit, the potentiometer is also coupled to the adjustment knob such that when the adjustment knob is rotated, it can act on the potentiometer.

2. The auto-darkening filter as recited in claim 1, wherein the adjustment knob comprises:
an outer knob in the form of a cap and at least partially exposed on the outer surface of the first shell body and be covered about the outer periphery of the annular projection;
an inner knob disposed in the interior of the outer shell, one side of the inner knob adjacent to the outer knob being connected to the outer knob such that coaxial rotation of the inner knob is driven by the outer knob; the other side of the inner knob away from the outer knob having a rotational shaft coupled to the potentiometer.

3. The auto-darkening filter as recited in claim 1, wherein one of the outer and inner knobs has at least a positioning projection, the other of the outer and inner knobs has at least a positioning groove for mating with the positioning projection, such that the positioning projection is insertable and securable in the positioning groove.

4. The auto-darkening filter as recited in claim 3, wherein the positioning groove is disposed in the inner knob and the positioning groove is embodied as multiple positioning grooves.

5. The auto-darkening filter as recited in any one of claims 2 to 4, wherein the inner knob at its outer contour is provided with a first limit part protruding therefrom, the first shell body at its inner surface is provided with a second limit part corresponding to the first limit part, when the first limit part is in contact with the second limit part, further rotation of the inner knob is impeded by the second limit part.

6. The auto-darkening filter as recited in claim 1, wherein a side of the adjustment knob adjacent to the annular projection is provided with an annular groove for mating with the annular projection, such that the annular projection can be received in the annular groove.

7. The auto-darkening filter as recited in claim 6, wherein the first shell body also comprises a first engagement part disposed on a circumferential inner wall of the annular projection and extending along a radial direction of the annular projection towards the center of the annular projection;
wherein the adjustment knob also comprises a second engagement part disposed adjacent to the annular groove, when the adjustment knob is installed, the second engagement part is snapped with the first engagement part.

8. The auto-darkening filter as recited in claim 7, wherein a side of the adjustment knob adjacent to the potentiometer is provided with a rotational shaft coupled to the potentiometer, an isolation part is formed in a portion between the rotational shaft and the annular groove, and the second engagement part is disposed at a side of the isolation part adjacent to the annular groove.

9. The auto-darkening filter as recited in any one of claims 6 to 8, wherein the adjustment knob also comprises a first limit part disposed in the annular groove, the first shell body also comprises a second limit part disposed on a top of the annular projection and for mating with the first limit part, when the first limit part is in contact with the second limit part, further rotation of the adjustment knob is impeded by the second limit part.

10. A welding helmet, comprising a helmet body and an auto-darkening filter as recited in any one of claims 1 to 9, wherein the helmet body can be worn by a head of a user, and the auto-darkening filter is releasably connected to the helmet body.
